(19) Europäisches Patentamt
European Patent Office
Office européen des brevets

(11) Veröffentlichungsnummer: **0 437 645 B1**

(12) # EUROPÄISCHE PATENTSCHRIFT

(45) Veröffentlichungstag der Patentschrift: **29.03.95**

(51) Int. Cl.[6]: **C07D 401/12**, A61K 31/415, A61K 31/44

(21) Anmeldenummer: **90100726.0**

(22) Anmeldetag: **15.01.90**

(54) **Imidazolylpropylguanidinderivat, Verfahren zu seiner Herstellung und diese Verbindung enthaltende Arzneimittel.**

(43) Veröffentlichungstag der Anmeldung:
**24.07.91 Patentblatt 91/30**

(45) Bekanntmachung des Hinweises auf die Patenterteilung:
**29.03.95 Patentblatt 95/13**

(84) Benannte Vertragsstaaten:
**AT BE CH DE DK ES FR GB GR IT LI LU NL SE**

(56) Entgegenhaltungen:
**EP-A- 0 199 845**
**DE-A- 3 726 381**

(73) Patentinhaber: **HEUMANN PHARMA GMBH & CO**
**Heideloffstrasse 18 - 28**
**D-90478 Nürnberg (DE)**

(72) Erfinder: **Mörsdorf, Peter, Dr. Dipl.-Chem.**
**Sportplatzstrasse 4**
**W-8506 Langenzenn (DE)**
Erfinder: **Engler, Heidrun, Dr. Dr. med. vet.**
**Ringstrasse 23**
**W-8501 Cadolzburg (DE)**
Erfinder: **Schickaneder, Helmut, Dr. Dipl.-Chem.**
**Wiesenstrasse 16**
**W-8501 Eckental (DE)**
Erfinder: **Ahrens, Kurt-Henning, Dr.**
**Trödelmarkt 42**
**W-8500 Nürnberg (DE)**

(74) Vertreter: **Kraus, Walter, Dr. et al**
**Patentanwälte Kraus, Weisert & Partner**
**Thomas-Wimmer-Ring 15**
**D-80539 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 437 645 B1

## Beschreibung

Die Herzinsuffizienz ist ein vor allem in den Industrieländern weit verbreitetes klinisches Syndrom, das durch die Unfähigkeit des Herzens gekennzeichnet ist, eine dem Bedarf des Organismus entsprechende Auswurfleistung zu erbringen. Digitalisglykoside und $\beta$-Sympathomimetika waren für viele Jahrzehnte die beiden wichtigsten Gruppen von Arzneistoffen, die zur medikamentösen Therapie der Herzinsuffizienz eingesetzt wurden. Ihre vielfältigen Nachteile jedoch führten in den vergangenen Jahren zu einer intensiven Suche nach neuen Wirkstoffklassen zur Behandlung der Herzinsuffizienz. Dabei wurden sowohl in der Gruppe der Phosphodiesterase-III-Hemmer als auch in den Histamin-$H_2$-Agonisten potente cardiotone Substanzen gefunden.

Die DE-OS'en 35 12 084, 35 28 214, 35 28 215 und 36 31 334 und die EP-OS 0 199 845 beschreiben Histamin-$H_2$-Agonisten, in denen einerseits die Wirkstärke der Verbindungen im Vergleich zu Impromidin (G.J. Durant et al., Nature (London), 1978, 276, 403) erheblich gesteigert werden konnte und andererseits durch den Einbau zusätzlicher $H_1$-antagonistischer Bausteine unerwünschte Nebenwirkungen des Impromidins beseitigt werden konnten. Jedoch sind diese Verbindungen wie Impromidin bei oraler Verabreichung nicht oder nur unzureichend wirksam, während sie bei parenteraler Applikation ausgezeichnete cardiotone Wirksamkeit aufweisen.

In der DE-OS 37 26 381 werden Guanidincarbonsäureester der allgemeinen Formel

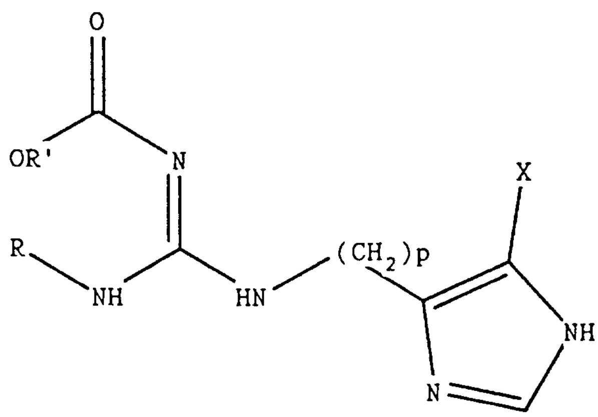

beschrieben, worin R, R' und X für bestimmte Gruppierungen stehen und p den Wert 2 oder 3 hat.

Wie in der DE-OS 37 26 381 beschrieben, weisen die Verbindungen der **obigen Formel** sehr gute kontraktilitätssteigernde Wirksamkeiten am Herzen bei erheblich verbesserter oraler Verfügbarkeit auf. Gegenüber den nicht mit einer Estergruppe substituierten Guanidinderivaten der oben genannten deutschen und europäischen Offenlegungsschriften ist die orale Verfügbarkeit ungefähr um den Faktor 20 höher. Jedoch fehlt diesen Verbindungen wiederum eine ausreichende parenterale, z. B. intravenöse Verfügbarkeit.

Es wurde nun gefunden, daß eine besondere Verbindung, die unter die allgemeine Formel der in der Deutschen Offenlegungsschrift DE-OS 37 26 381 beschriebenen und beanspruchten Verbindungen fällt, welche aber nicht speziell darin beschrieben ist, besondere unvorhersehbare Vorteile aufweist. Diese Verbindung weist eine Kombination von besonders vorteilhaften Eigenschaften für die Behandlung von Erkrankungen des Herzens und des Kreislaufs auf.

Gegenstand der vorliegenden Erfindung ist daher die Verbindung $N^1$-[3-(3,4-Difluorphenyl)-3-(2-pyridyl)-propyl]-$N^2$-tert.-butoxycarbonyl-$N^3$-[3-(1H-imidazol-4-yl)propyl]-guanidin der Formel I

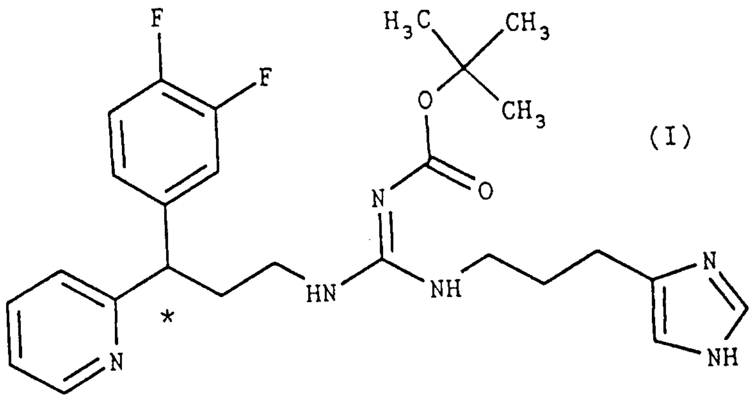

(I)

und ihre physiologisch annehmbaren Salze und Solvate. Die Verbindung der **Formel I** kann in einer Reihe von tautomeren Formen, wie dies durch die **Formeln Ia - If** wiedergegeben ist, und in zwei enantiomeren Formen bezüglich des optisch aktiven Kohlenstoffatoms C* vorliegen. Gegenstand der Erfindung sind daher auch die enantiomeren und tautomeren Derivate der Verbindung der **Formel I**.

Die Verbindung der **Formel I** ist pharmakologisch charakterisiert durch ein duales Wirkprofil, in dem sie sowohl hohe Histamin-$H_2$-agonistische als auch $H_1$-antagonistische Wirkung besitzt. Die Kombination dieser beiden Eigenschaften ist bei der Behandlung von Erkrankungen des Herzens und des Kreislaufs besonders vorteilhaft, da einerseits die hohe $H_2$-agonistische Aktivität zu einer guten positiv inotropen Wirkung führt und andererseits die $H_1$-antagonistische Komponente schädliche Nebenwirkungen, wie z. B. Koronarspasmen verhindert.

Gegenüber den in der DE-OS 37 26 381 konkret beschriebenen Verbindungen besitzt die erfindungsgemäße Verbindung der Formel I zwei wesentliche Vorteile. In-vivo-Versuche mit Meerschweinchen haben gezeigt, daß die Verbindung der Formel I nicht nur bei oraler Verabreichung, sondern auch bei parenteraler Verabreichung, z.B. durch intravenöse Infusion, eine ausgezeichnete positiv inotrope Wirkung besitzt (Tabelle I).

Dies ermöglicht die Verwendung der Verbindung sowohl in der Notfallmedizin zur Behandlung der akuten Herzinsuffizienz als auch zur oralen Dauertherapie der chronischen Herzinsuffizienz.

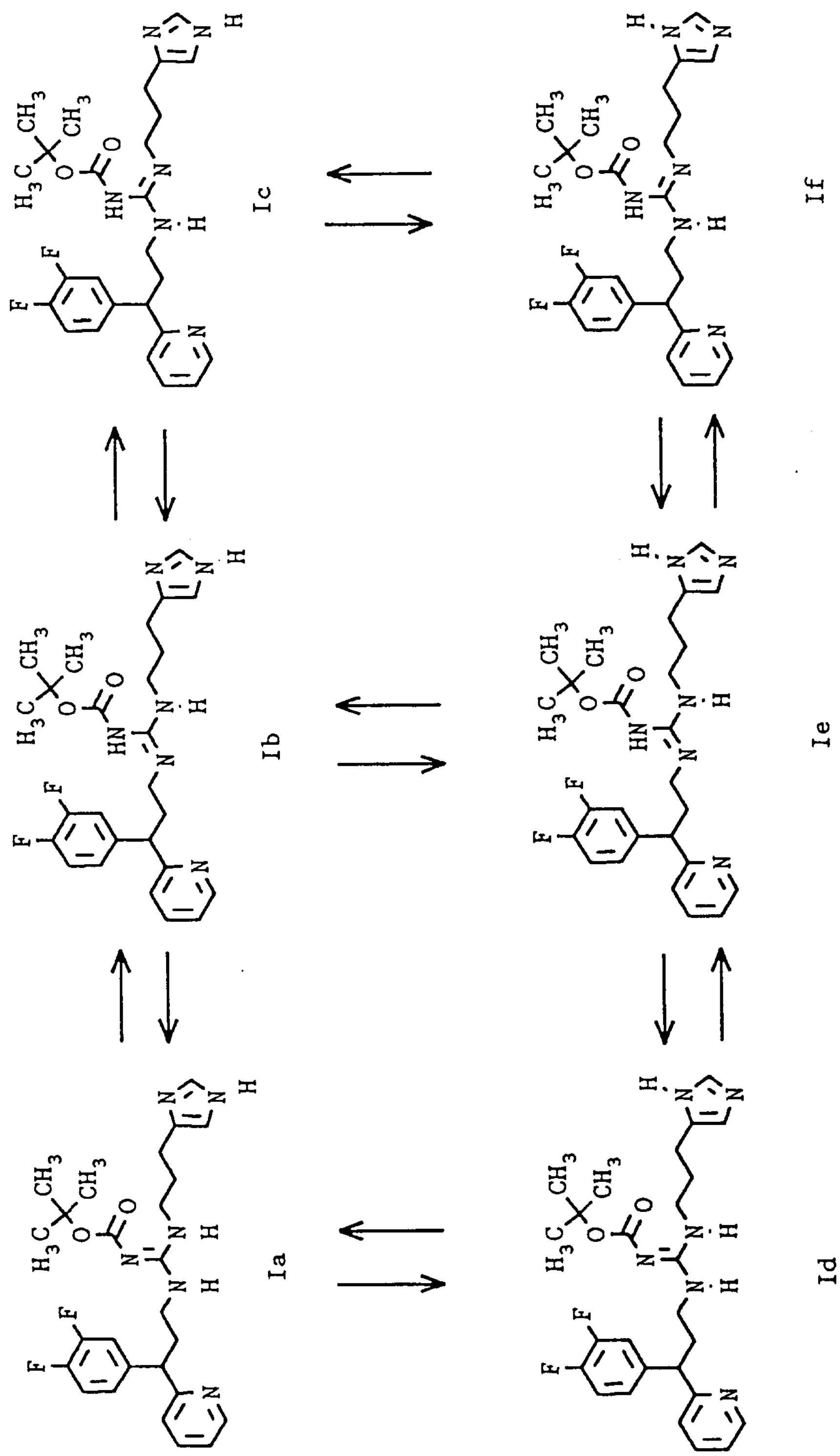

Ic If Ib Ie Ia Id

Hämodynamische Charakterisierung am narkotisierten Meerschweinchen

Methode:

Die Tiere werden mit Urethan (1,5g/kg) narkotisiert. Zur volumenkontrollierten Beatmung wird die Trachea kanüliert. Danach erfolgt die operative Freilegung beider Karotiden; über die rechte Karotis wird ein TIP-Katheder (3F) eingeführt, der unter fortlaufender Druckregistrierung dann über die Aorta ascendens in den linken Ventrikel vorgeführt wird. Die erfolgreiche Passage der Aortenklappen wird hierbei durch die typische linksventrikuläre Druckkurve erkannt. Über die linke Karotis wird zur Thermodilution ein Thermistorfühler (3F, F. Edwards) in den Aortenbogen vorgeschoben. Der Thermistorfühler hat gleichzeitig ein Lumen zur arteriellen Blutdruckregistrierung. Zur Applikation des Kälteinjekts (0,2 ml 0,9% NaCl, 15 °C) wird durch die rechte Vena jugularis ein Katheder vor den rechten Vorhof plaziert.

Die Applikation der Testsubstanzen erfolgt nach hämodynamischer Stabilisierung und unter β-Blockade (Metoprolol 2 mg/kg i.m.). Alle Kreislaufparameter werden kontinuierlich auf einem Direktschreiber registriert. Die Kontraktilität (dp/dt) wird über die Volumenkurve berechnet.

Zur intravenösen Applikation werden die Testsubstanzen in physiologischer Kochsalzlösung gelöst und über die linke Vena jugularis infundiert (Infusionsvolumen 0,02 ml/min). In den Versuchen zur oralen Verfügbarkeit wird das Duodenum mittels eines 1 cm langen Medianschnitts im oberen Abdominalbereich freigelegt und die Testsubstanzen über eine Nadel in das Duodenum injiziert. Die Substanzen sind dazu in Tylose (Injektionsvolumen 1 ml/kg) suspendiert.

Tabelle I

| Positiv inotrope Wirkung in vivo am narkotisierten Meerschweinchen nach intravenöser bzw. intraduodenaler Verabreichung | | | | |
|---|---|---|---|---|
| **Substanz** | **Anstieg von dp/dt in Prozent** | | | |
| | intravenöse Applikation | | intraduodenale Applikation | |
| | Dosis µg/kg/min | dp/dt (Anstieg %) x ± sd(n = 3) | Dosis mg/kg | dp/dt (Anstieg %) x ± sd(n = 3) |
| Beispiel 1 | 0,6<br>1,2 | 41,8 ± 13,4<br>146,4 ± 16,7 | 3,125<br>6,25 | 56,6 ± 14,9<br>165,0 ± 10,8 |
| Vergleich 1 | 10 | 45,8 ± 4,5 | 3,125<br>6,25 | 54,2 ± 21,4<br>123,8 ± 42,6 |
| Vergleich 2 | 2,5<br>5<br>10 | 22,7 ± 11,6<br>70,8 ± 12,3<br>110,6 ± 9,7 | 6,25<br>12,5<br>25,0 | 33,4 ± 16,7<br>93,9 ± 18,5<br>192,8 ± 24,6 |
| Vergleich 3 | 10 | 33,2 ± 18,7 | 3,125<br>6,25 | 67,3 ± 15,8<br>154,8 ± 26,6 |
| Vergleich 4 | 5<br>10<br>20 | 10,8 ± 7,6<br>21,4 ± 8,4<br>25,7 ± 4,6 | 12,5<br>18,8<br>25,0 | 27,2 ± 12,5<br>75,7 ± 18,8<br>158,5 ± 43,6 |
| Vergleich 5 | 10 | 21,6 ± 13,4 | 3,125<br>6,25<br>12,5 | 22,2 ± 9,7<br>69,8 ± 12,4<br>211,3 ± 42,9 |
| Vergleich 6 | 2,0 | 24,6 ± 9,4 | 3,125<br>6,25 | 62,4 ± 18,2<br>131,3 ± 15,4 |

**Tabelle 2** Chemische Strukturen der Vergleichsverbindungen

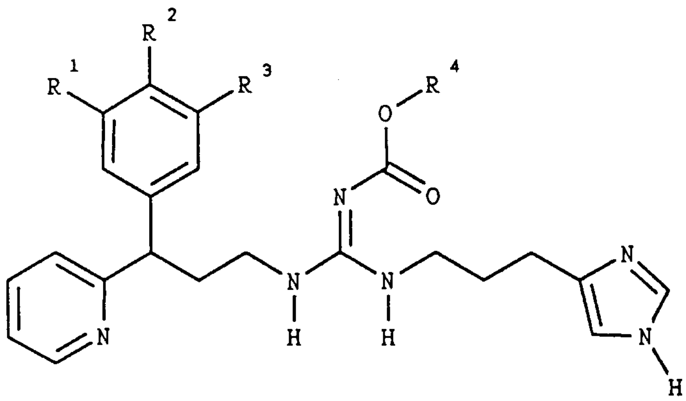

| | $R^1$ | $R^2$ | $R^3$ | $R^4$ |
|---|---|---|---|---|
| Vergleich 1[a] | F | F | H | $C_2H_5$ |
| Vergleich 2[b] | F | H | F | $CH_3$ |
| Vergleich 3[c] | F | H | F | $C_2H_5$ |
| Vergleich 4[d] | F | H | F | $n\text{-}C_4H_9$ |
| Vergleich 5 | F | H | F | $t\text{-}C_4H_9$ |
| Vergleich 6[e] | H | F | H | $t\text{-}C_4H_9$ |

[a-e]: *Verbindungen aus der DE-OS 37 26 381:*
[a]: *Beispiel 13*
[b]: *Beispiel 24*
[c]: *Beispiel 14*
[d]: *Beispiel 25*
[e]: *Beispiel 16*

Eine wesentliche Nebenwirkung von Histamin-$H_2$-Agonisten ist, bedingt durch das Vorhandensein von Histamin-$H_2$-Rezeptoren im Magen, die Stimulation der Magensäuresekretion. Dies schränkt die Verwendbarkeit der Verbindungen zur Behandlung von Herz-Kreislauf-Erkrankungen ein, da es bei höherer Dosierung oder längerer Anwendung zu Ulcera der Magenschleimhaut kommen kann. Überraschenderweise wurde gefunden, daß die Verbindung der **Formel I** in in vivo-Versuchen an Meerschweinchen zu keiner relevanten Stimulierung der Säuresekretion führt.

Bestimmung der Säuresekretion am Meerschweinchen in vivo

Methode:

Zur Bestimmung der Magensäuresekretion in vivo am Meerschweinchen wurde die Methode von Gosh und Schild (Ratte) modifiziert für Meerschweinchen.

Männliche Meerschweinchen mit einem Körpergewicht von ca. 300g werden mit Urethan (2g/kg i.m.) betäubt. Die Bauchhöhle wird eröffnet und ein Polyethylenschlauch durch die Speiseröhre in den Magen eingeführt. Durch das Duodenum wird ein zweiter Schlauch in den Magen eingeführt. Dieser Schlauch ist mit einem 10 ml fassenden Ballon versehen, der mit Luft aufgeblasen wird, um das Volumen des nichtsekretierenden Magenlumens zu reduzieren. Zur Vermeidung einer Kontamination des Magensafts mit Gallenflüssigkeit wird der Gallengang katheterisiert.

Der Magen wird mit einer vorgewärmten Kochsalzlösung (15 ml/10 min) perfundiert und das Perfusat in 10-Minuten-Intervallen gesammelt. Die sekretierte Säuremenge in den Proben wird durch Titration gegen 0,1 N NaOH mit Hilfe eines automatischen Titrators (ABU 80 Radiometer) bestimmt.

Bei parenteraler Applikation werden die Testsubstanzen wiederum mittels eines Polyethylen-Katheters, der in der linken Vena jugularis liegt, verabreicht.

Tabelle 3

| Bestimmung der Magensäuresekretion am narkotisierten Meerschweinchen | | |
|---|---|---|
| Testsubstanz | Zunahme der Säuresekretion | |
| | Dosis µg/kg/min | HCl µeq/ml |
| basale Sekretion | - | 31,8 ± 20,2 |
| Beispiel 1 | 0,3<br>0,6<br>1,2<br>2,4 | 22,6 ± 9,7<br>83,7 ± 10,1<br>71,6 ± 10,5<br>31,2 ± 8,4 |
| Vergleich 6 | 2,5 | 122,6 ± 32,3 |
| Vergleich A[a)] | 0,03<br>0,06<br>0,12 | 12,1 ± 5,0<br>41,5 ± 10,8<br>119,2 ± 15,9 |
| Vergleich B[b)] | 0,015<br>0,030<br>0,060<br>0,125<br>0,250 | 46,8 ± 10,2<br>53,0 ± 10,3<br>86,3 ± 11,1<br>112,1 ± 28,7<br>121,4 ± 14,8 |
| Impromidin | 0,015<br>0,030<br>0,060<br>0,120 | 27,6 ± 9,5<br>52,9 ± 21,1<br>126,2 ± 28,0<br>116,0 ± 39,3 |

[a)] *EP – OS 0199 845, Beispiel 133*
[b)] *DE – OS 3631 334, Beispiel 17*

Die erfindungsgemäße Verbindung der Formel I kann nach zwei verschiedenen Verfahrensvarianten hergestellt werden:

1.) durch Umsetzung einer Verbindung der **Formel II**

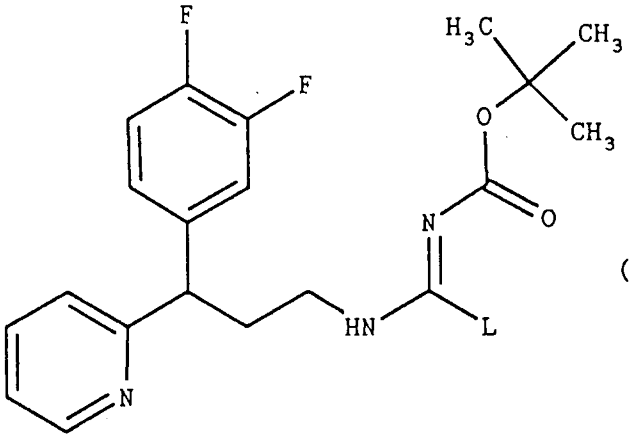

(II)

in der L für eine $C_1$-$C_4$-Alkylthio-, $C_1$-$C_4$-Alkoxy-, Arylthio- oder Aryloxygruppe, vorzugsweise eine Methylthio- oder Phenoxygruppe steht, mit einer Verbindung der **Formel III**

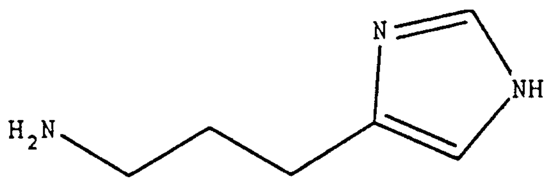

(III)

oder
2.) durch Umsetzung einer Verbindung der **Formel IV**

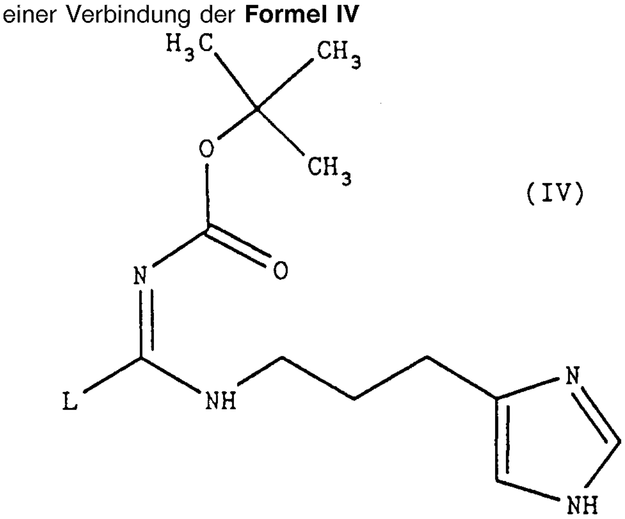

(IV)

in der L die gleiche Bedeutung hat wie in der Verfahrensvariante 1, mit einer Verbindung der **Formel V**

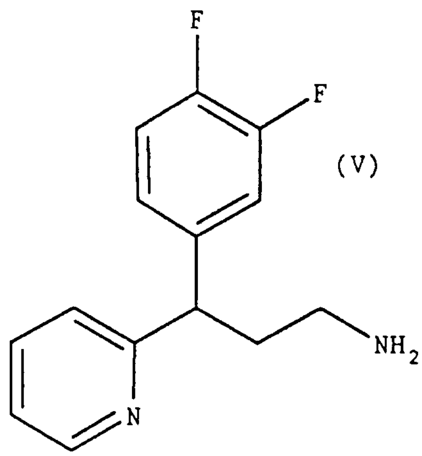

(V)

Die Umsetzungen erfolgen vorzugsweise in äquimolaren Mengen und in einem polaren Lösungsmittel, wie z. B. Acetonitril, Dimethylsulfoxid, Dimethylformamid oder Pyridin, vorzugsweise in Acetonitril, bei Temperaturen von Raumtemperatur bis zur Rückflußtemperatur des verwendeten Lösungsmittel.

Die Herstellung der in beiden Verfahrensvarianten eingesetzten Ausgangsverbindungen der **Formeln II** und **IV** erfolgt analog zu den in der DE-OS 37 26 381 beschriebenen Methoden.

Die nach den beiden Verfahrensvarianten erhaltene Verbindung der **Formel I** wird in üblicher Weise isoliert und gereinigt, beispielsweise durch chromatographische Arbeitsweisen, Umkristallisation etc.

Die Verbindung der **Formel I** kann gegebenenfalls in eines ihrer physiologisch annehmbaren Salze umgewandelt werden. Die Erfindung umfaßt daher auch die physiologisch annehmbaren Salze der Verbindung.

Diese Salze können z. B. mit Mineralsäuren, wie Chlor-, Brom-, Jodwasserstoffäure, Phosphorsäure, Metaphosphorsäure, Salpetersäure oder Schwefelsäure, oder mit organischen Säuren, wie Ameisensäure, Essigsäure, Propionsäure, Phenylessigsäure, Weinsäure, Zitronensäure, Fumarsäure, Methansulfonsäure, Embonsäure etc. nach an sich bekannten Methoden gebildet werden.

Die Verbindung der Formel I kann gegebenenfalls in eines ihrer physiologisch annehmbaren Solvate umgewandelt werden. Die Erfindung umfaßt daher auch die physiologisch annehmbaren Solvate der Verbindung.

Diese Solvate können zum Beispiel mit Wasser, Alkoholen, wie Methanol, Ethanol, Isopropanol, n-Butanol, oder Ketonen, wie Aceton oder Ethylmethylketon, hergestellt werden.

Die Herstellung der Solvate erfolgt zweckmäßig durch Auflösen der Verbindung der Formel I in den gegebenen Lösungsmitteln und Kristallisation der Solvate oder durch Eindampfen der Lösungen im Vakuum.

Die erfindungsgemäßen Verbindungen können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung eines oder mehrerer pharmazeutisch annehmbarer Träger oder Verdünnungsmitteln hergestellt werden.

Die erfindungsgemäßen Verbindungen können daher für die orale, bukkale, topische, parenterale oder rektale Verabreichung formuliert werden.

Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind.

Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/oder Dispergierungsmittel, enthalten.

Alternativ kann der Wirkstoff auch in Pulverform zur Rekonstitution mit einem geeigneten Träger, z. B. sterilem, pyrogenfreiem Wasser, vor dem Gebrauch vorliegen. Die erfindungsgemäßen Verbindungen

können auch für rektale Zubereitungen, z. B. Suppositorien oder Retentionseinläufe, formuliert werden, die z. B. herkömmliche Suppositoriengrundlagen, wie Kakaobutter oder andere Glyceride, enthalten. Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis insgesamt 5 mg bis 1 g/Tag, je nach Zustand des Patienten.

Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es Fälle, in denen mit weniger als der oben genannten Mindestmenge ausgekommen werden kann, während in anderen Fällen die genannte obere Grenze überschritten werden muß.

Beispiel 1

**N[1]-[3-(3,4-Difluorphenyl)-3-(2-pyridyl)-propyl]-N[2]-tert.-butoxycarbonyl-N[3]-[3-(1H-imidazol-4-yl)propyl]-guanidin**

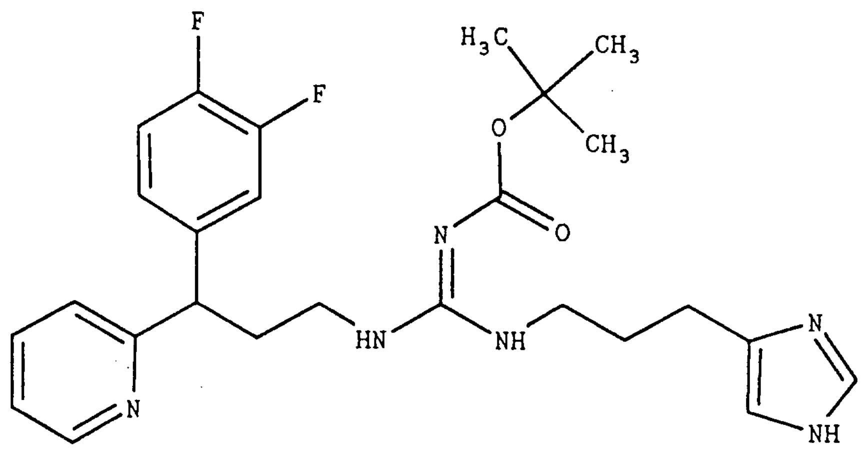

**a) N[1]-[3-(3,4-Difluorphenyl)-3-(2-pyridyl)-propyl]-N[2]-tert.-butoxycarbonyl-O-phenyl-isoharnstoff**

5,0g (20,1 mmol) 3-(3,4-Difluorphenyl)-3-(2-pyridyl)-propylamin in 50 ml Acetonitril werden mit 6,3g (20,1 mmol) N-tert.-Butoxycarbonyl-O,O-diphenyl-imidocarbonat versetzt und eine Stunde bei Raumtemperatur gerührt.
Eine dünnschichtchromatographische Kontrolle des Reaktionsgemisches auf DC-Folien Polygram SIL $G/UV_{254}$ (Macherey-Nagel) mit Dichlormethan:Methanol 99:1 als Eluent zeigt vollständige Umsetzung an. Das Produkt wird weder isoliert noch gereinigt, sondern direkt weiter umgesetzt.

$C_{26}H_{27}F_2N_3O_3$ (467,52)

**b) N[1]-[3-(3,4-Difluorphenyl)-3-(2-pyridyl)-propyl]-N[2]-tert.-butoxycarbonyl-N[3]-[3-(1H-imidazol-4-yl)-propyl]-guanidin**

Zu dem unter a) erhaltenen Reaktionsgemisch werden 2,5g (20,1 mmol) 3-(1H-Imidazol-4-yl)-propylamin gegeben.
Danach wird die Lösung 13 Stunden unter Rückfluß gekocht. Nach Abdampfen des Lösungsmittels im Wasserstrahlvakuum erhält man ein gelbes Öl, das an 250g Kieselgel 60 (Merck Nr. 7734) mit Dichlormethan:Methanol 90:10 als Laufmittel chromatographiert wird.
Die Fraktionen mit Rf-Wert 0,2 werden vereinigt und im Vakuum eingedampft. Das verbleibende farblose Öl kristallisiert beim Verrühren mit tert.-Butylmethylether/Ethylacetat (2:1). Es werden 2,8g (28%) farblose Kristalle vom Schmp. 94-95 °C erhalten.

$C_{26}H_{32}F_2N_6O_2$ (498,58)

$^1$H-NMR-Daten (DMSO-$d_6$, TMS als interner Standard)

δ = 1,37 (s) 9H
1,74 (quin) 2H
2,15-2,55 (m) 4H
3,0-3,25 (m) 4H
4,19 (t) 1H
6,78 (s) 1H
7,15-7,48 (m) 6H,
1H austauschbar mit $D_2O$
7,53 (s) 1H
7,70 (t) 1H
8,53 (d) 1H
8,9 (breit) 1H,
austauschbar mit $D_2O$ ppm.

**Patentansprüche**

**Patentansprüche für folgende Vertragsstaaten : DE, DK, GB, FR, IT, NL, SE, LI, CH, BE, AT, LU**

1. Imidazolylpropylguanidinderivat der **Formel I**

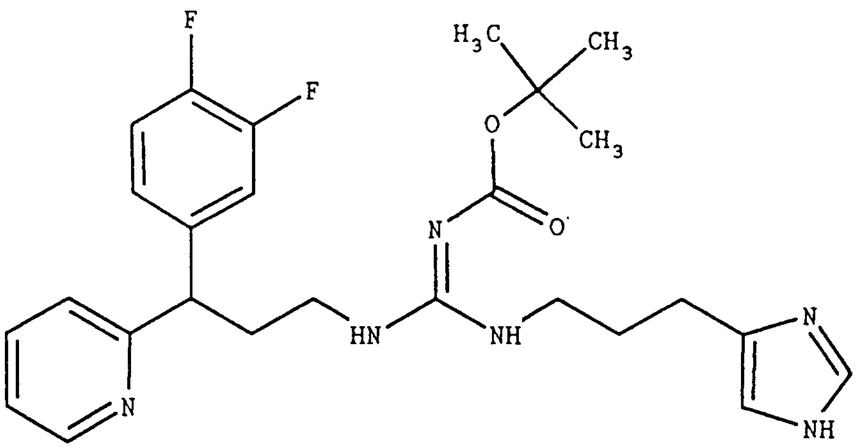

und seine physiologisch annehmbaren Salze und Solvate.

2. Verbindung nach Anspruch 1, dadurch gekennzeichnet, daß das physiologisch annehmbare Salz ein Säureadditionssalz mit einer organischen oder anorganischen Säure ist.

3. Verfahren zur Herstellung der Verbindung der **Formel I** gemäß Anspruch 1, dadurch gekennzeichnet, daß man

(a) eine Verbindung der **Formel II**

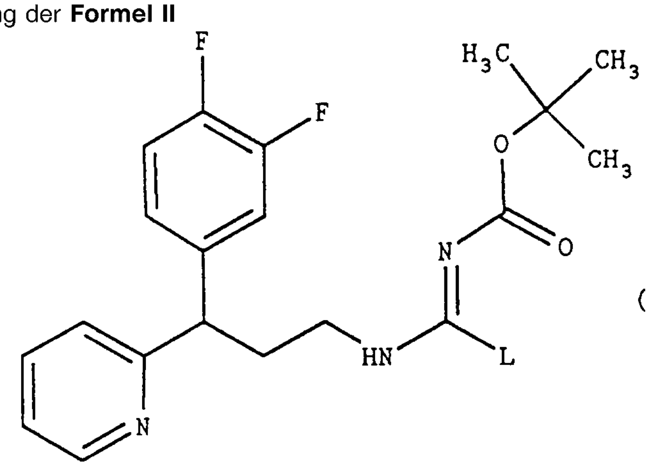

(II)

in der L für eine $C_1$-$C_4$-Alkylthio-, $C_1$-$C_4$-Alkoxy-, Arylthio- oder Aryloxygruppe steht, mit einer Verbindung der **Formel III**

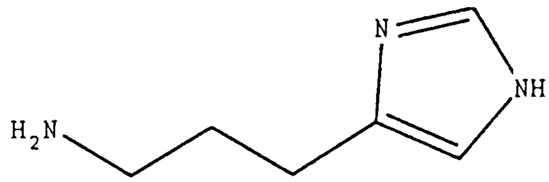

(III)

umsetzt, oder

(b) eine Verbindung der **Formel IV**

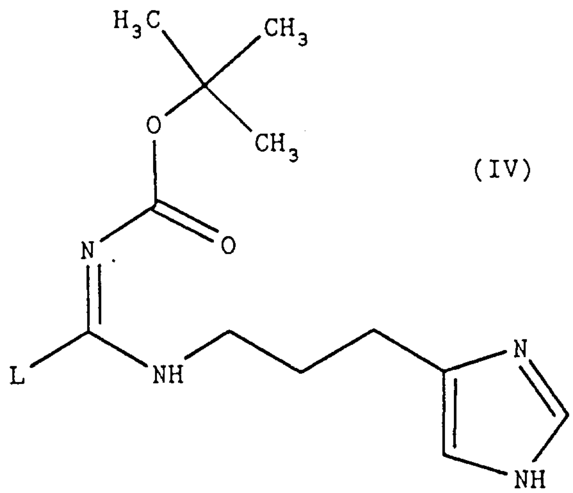

(IV)

in der L die oben angegebene Bedeutung hat, mit einer Verbindung der **Formel V**

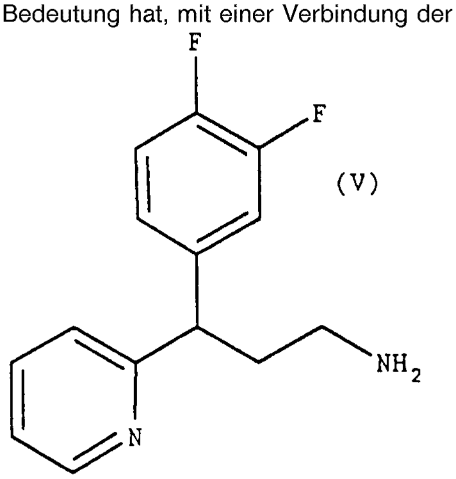

(V)

umsetzt,

und gegebenenfalls die nach (a) oder (b) erhaltene Verbindung der **Formel I** in an sich bekannter Weise in ihr physiologisch annehmbares Salz oder Solvat umwandelt.

**4.** Arzneimittel, dadurch gekennzeichnet, daß es mindestens eine Verbindung nach Anspruch 1 oder 2 zusammen mit einem inerten pharmazeutisch annehmbaren Träger oder einem inerten pharmazeutisch annehmbaren Verdünnungsmittel enthält.

**Patentanspruch für folgenden Vertragsstaat : ES**

**1.** Verfahren zur Herstellung eines Imidazolylpropylguanidinderivats der Formel I

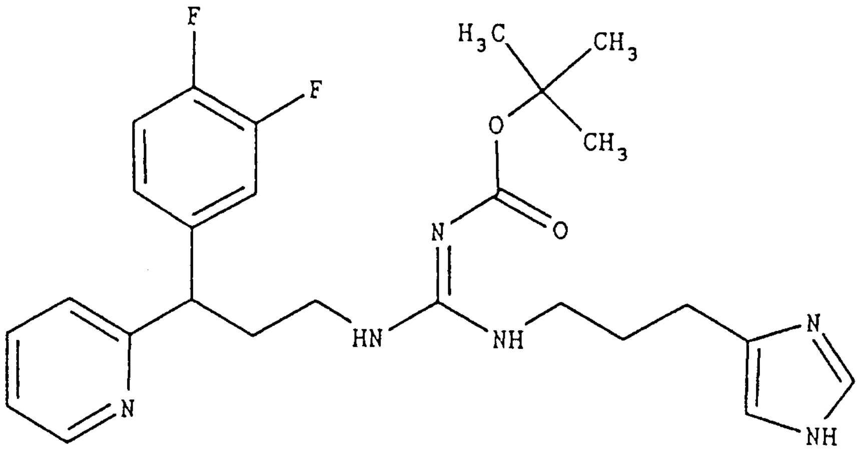

und seiner physiologisch annehmbaren Salze und Solvate, dadurch gekennzeichnet, daß man

(a) eine Verbindung der **Formel II**

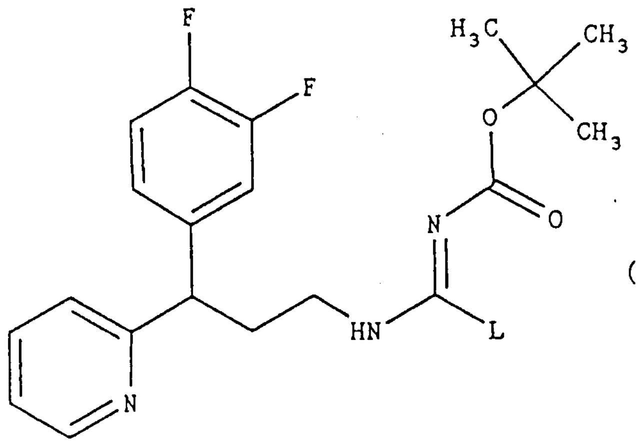

(II)

in der L für eine $C_1$-$C_4$-Alkylthio-, $C_1$-$C_4$-Alkoxy-, Arylthio- oder Aryloxygruppe steht, mit einer Verbindung der **Formel III**

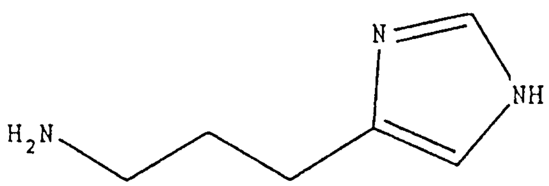

(III)

umsetzt, oder

(b) eine Verbindung der **Formel IV**

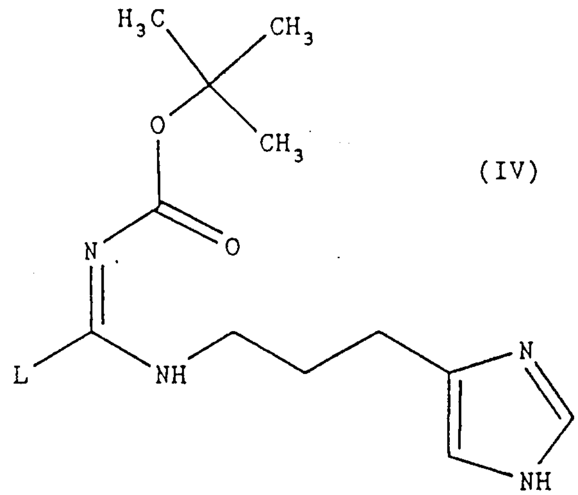

(IV)

in der L die oben angegebene Bedeutung hat, mit einer Verbindung der **Formel V**

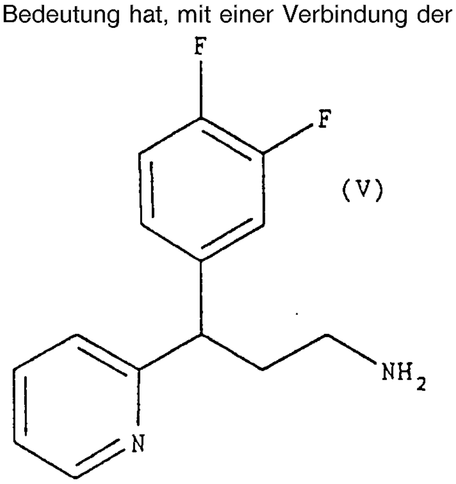

(V)

umsetzt,

und gegebenenfalls die nach (a) oder (b) erhaltene Verbindung der **Formel I** in an sich bekannter Weise in ihr physiologisch annehmbares Salz oder Solvat umwandelt.

**Patentansprüche für folgenden Vertragsstaat : GR**

**1.** Imidazolylpropylguanidinderivat der **Formel I**

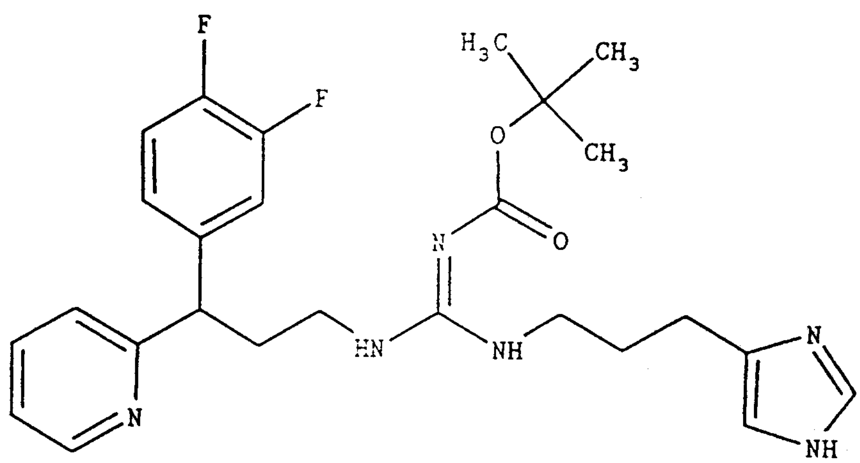

und seine Salze und Solvate.

**2.** Verbindung nach Anspruch 2, dadurch gekennzeichnet, daß das Salz ein Säureadditionssalz mit einer organischen oder anorganischen Säure ist.

**3.** Verfahren zur Herstellung der Verbindung der **Formel I** gemäß Anspruch 1, dadurch gekennzeichnet, daß man

(a) eine Verbindung der **Formel II**

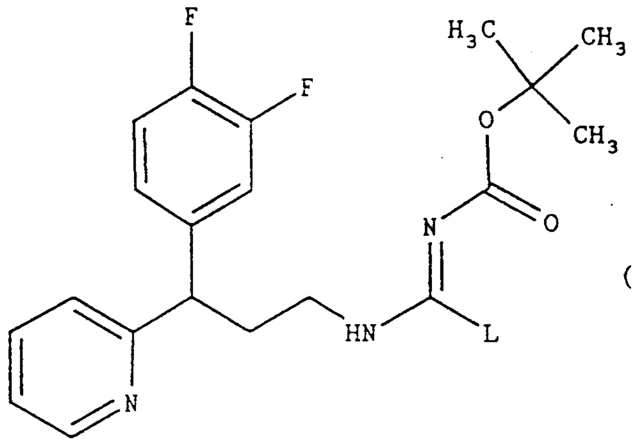

(II)

in der L für eine $C_1$-$C_4$-Alkylthio-, $C_1$-$C_4$-Alkoxy-, Arylthio- oder Aryloxygruppe steht, mit einer Verbindung der **Formel III**

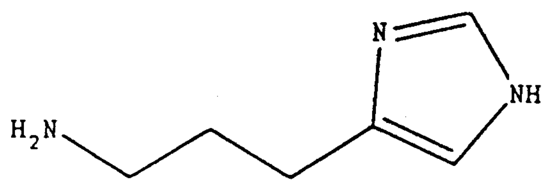

(III)

umsetzt, oder

(b) eine Verbindung der **Formel IV**

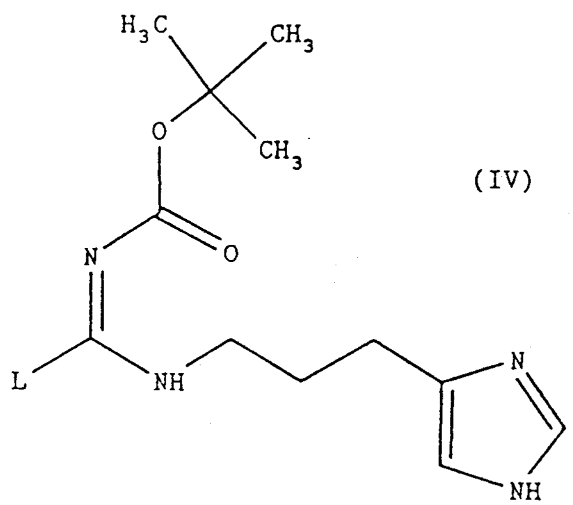

(IV)

in der L die oben angegebene Bedeutung hat, mit einer Verbindung der **Formel V**

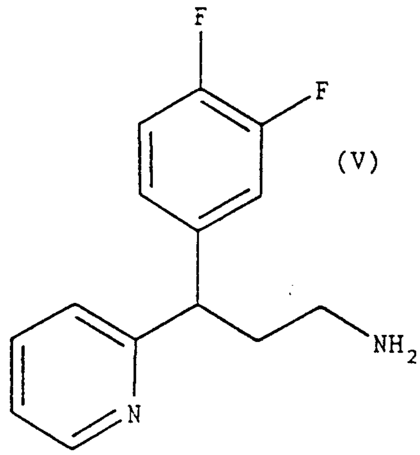

(V)

umsetzt,

und gegebenenfalls die nach (a) oder (b) erhaltene Verbindung der **Formel I** in an sich bekannter Weise in ihr physiologisch annehmbares Salz oder Solvat umwandelt.

## Claims

### Claims for the following Contracting States : DE, DK, GB, FR, IT, NL, SE, LI, CH, BE, AT, LU

**1.** An imidazolyl propyl guanidine derivative corresponding to formula I:

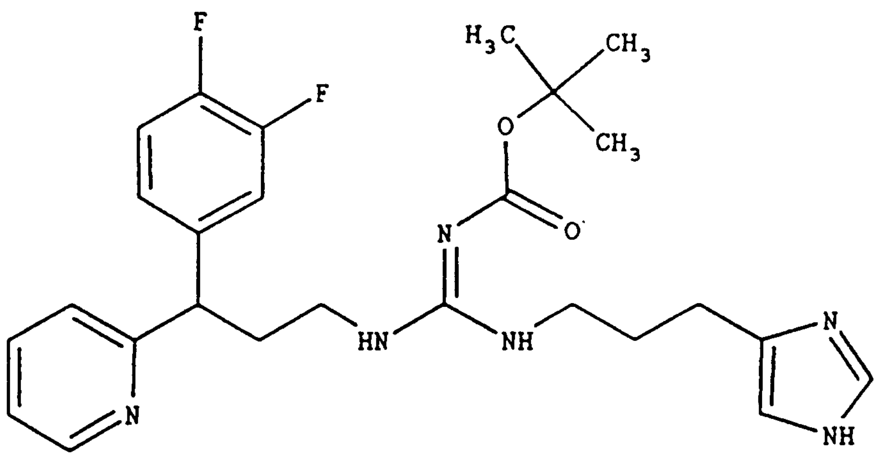

and physiologically acceptable salts and solvates thereof.

**2.** The compound claimed in claim 2, characterized in that the physiologically acceptable salt is an acid addition salt with an organic or inorganic acid.

**3.** A process for the production of the compound corresponding to formula I claimed in claim 1, characterized in that

(a) a compound corresponding to formula II:

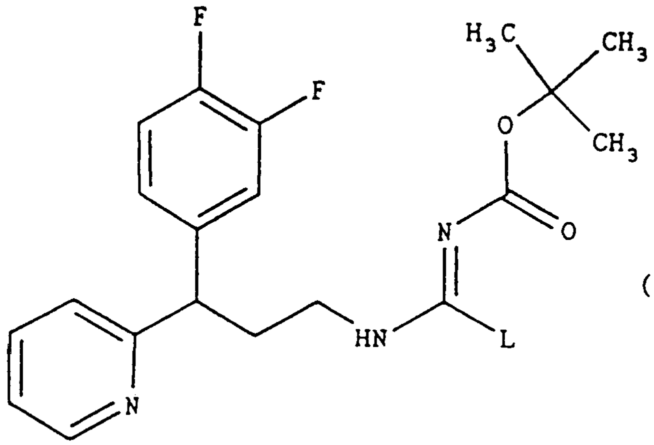

(II)

in which L is a $C_{1-4}$ alkylthio, $C_{1-4}$ alkoxy, arylthio or aryloxy group, is reacted with a compound corresponding to formula III:

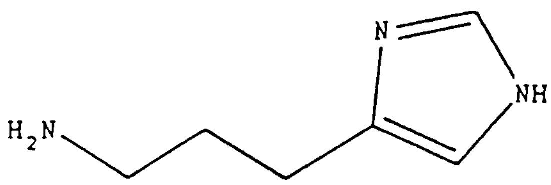

(III)

or

(b) a compound corresponding to formula IV:

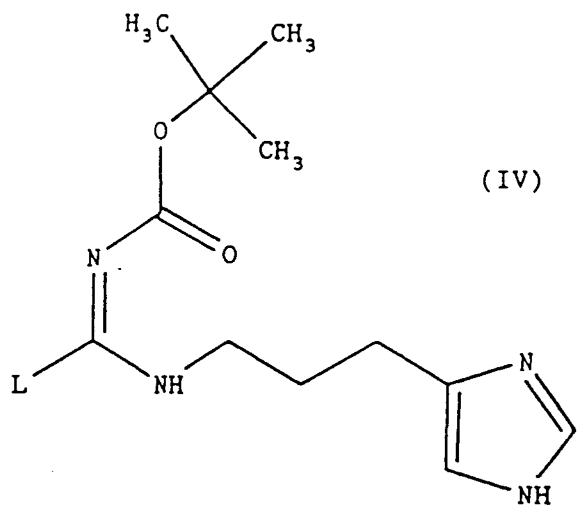

(IV)

in which L is as defined above, is reacted with a compound corresponding to formula V:

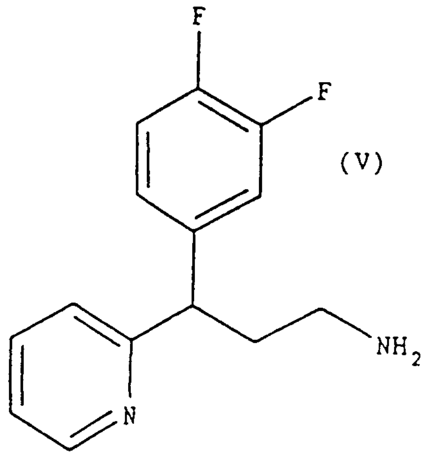

(V)

and the compound of formula (I) obtained in accordance with (a) or (b) is optionally converted in known manner into a physiologically acceptable salt or solvate.

4. A medicament, characterized in that it contains at least one compound according to claim 1 or 2 together with an inert pharmaceutically acceptable support or an inert pharmaceutically acceptable diluent.

**Claim for the following Contracting State : ES**

1. A process for the production of an imidazolyl propyl guanidine derivative corresponding to formula I:

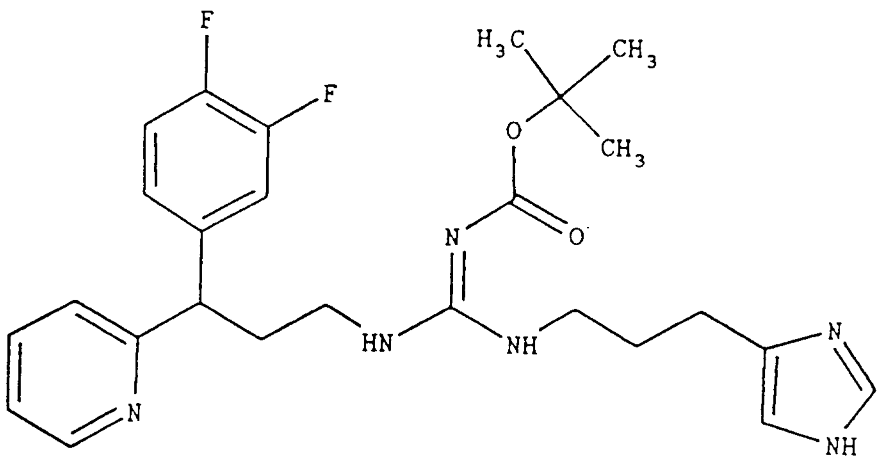

and physiologically acceptable salts and solvates thereof, characterized in that

(a) a compound corresponding to formula II:

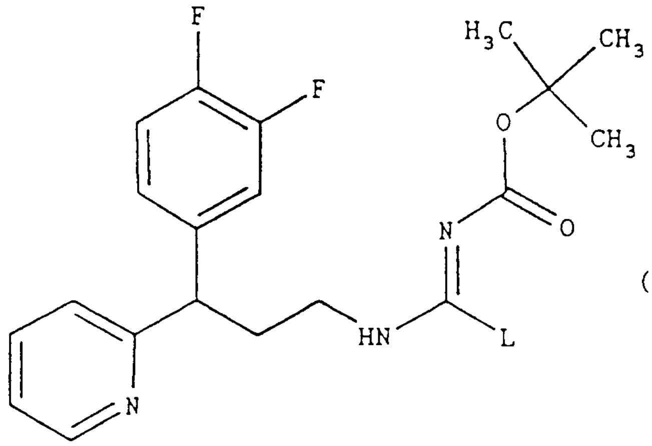

(II)

in which L is a $C_{1-4}$ alkylthio, $C_{1-4}$ alkoxy, arylthio or aryloxy group, is reacted with a compound corresponding to formula III:

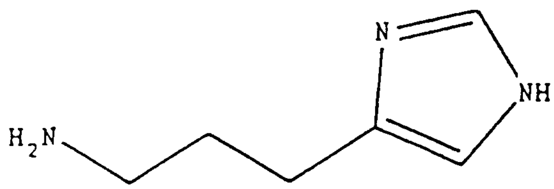

(III)

or

(b) a compound corresponding to formula IV:

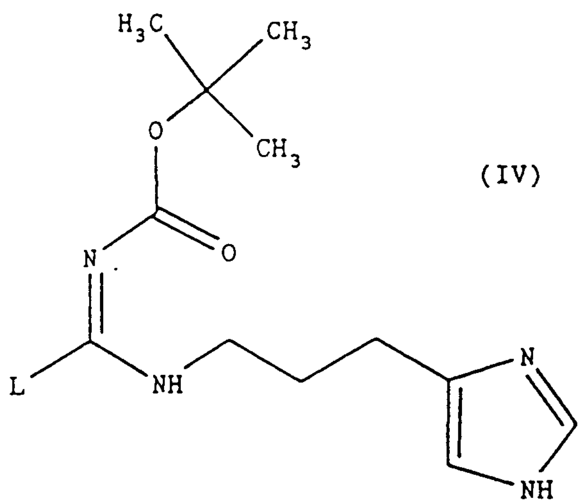

(IV)

in which L is as defined above, is reacted with a compound corresponding to formula V:

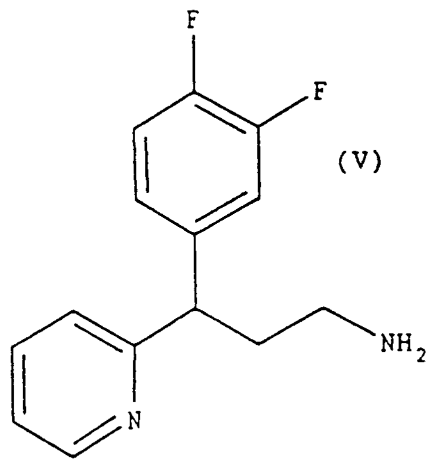

(V)

and the compound of formula (I) obtained in accordance with (a) or (b) is optionally converted in known manner into a physiologically acceptable salt or solvate.

**Claims for the following Contracting State : GR**

**1.** An imidazolyl propyl guanidine derivative corresponding to formula I:

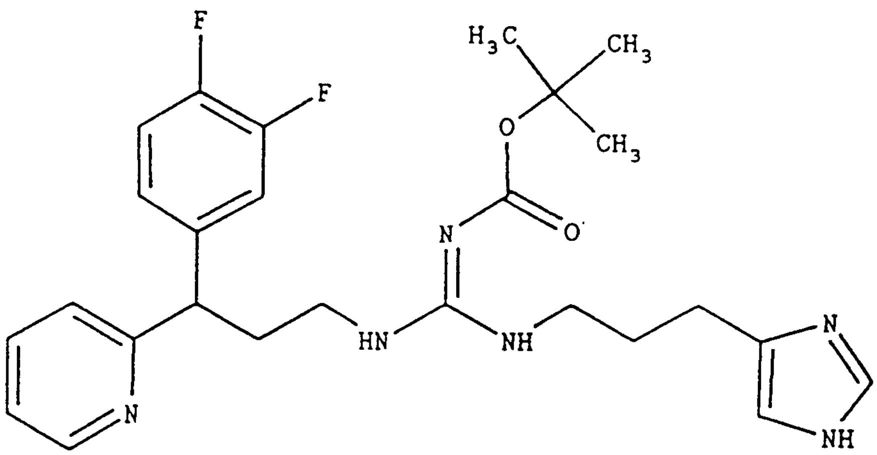

and salts and solvates thereof.

**2.** The compound claimed in claim 2, characterized in that the salt is an acid addition salt with an organic or inorganic acid.

**3.** A process for the production of the compound corresponding to formula I claimed in claim 1, characterized in that
(a) a compound corresponding to formula II:

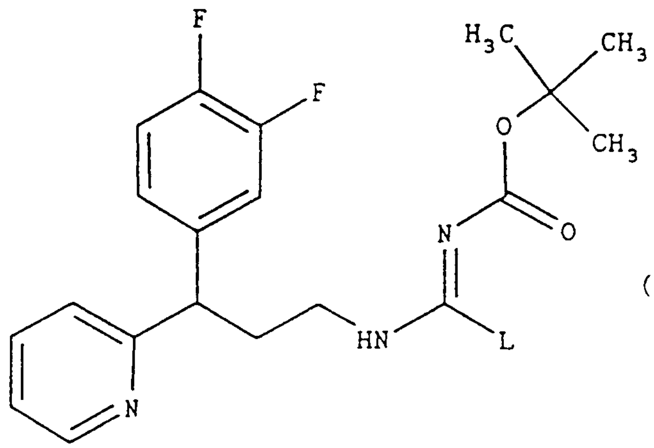

(II)

in which L is a $C_{1-4}$ alkylthio, $C_{1-4}$ alkoxy, arylthio or aryloxy group, is reacted with a compound corresponding to formula III:

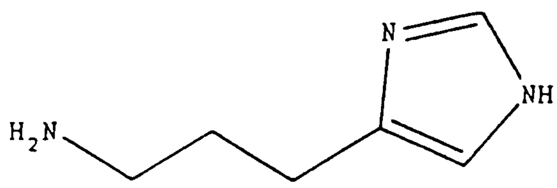

(III)

or

(b) a compound corresponding to formula IV:

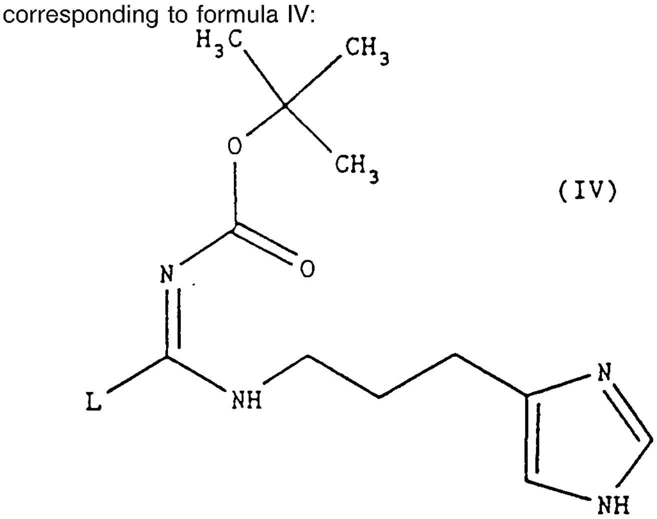

(IV)

in which L is as defined above, is reacted with a compound corresponding to formula V:

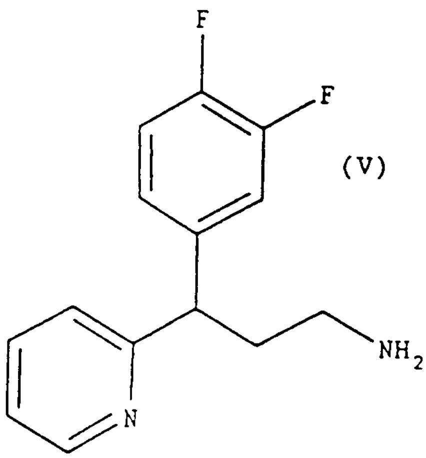

(V)

and the compound of formula (I) obtained in accordance with (a) or (b) is optionally converted in known manner into a physiologically acceptable salt or solvate.

**Revendications**

**Revendications pour les Etats contractants suivants : DE, DK, GB, FR, IT, NL, SE, LI, CH, BE, AT, LU**

1. Dérivé d'imidazolylpropylguanidine répondant à la formule I :

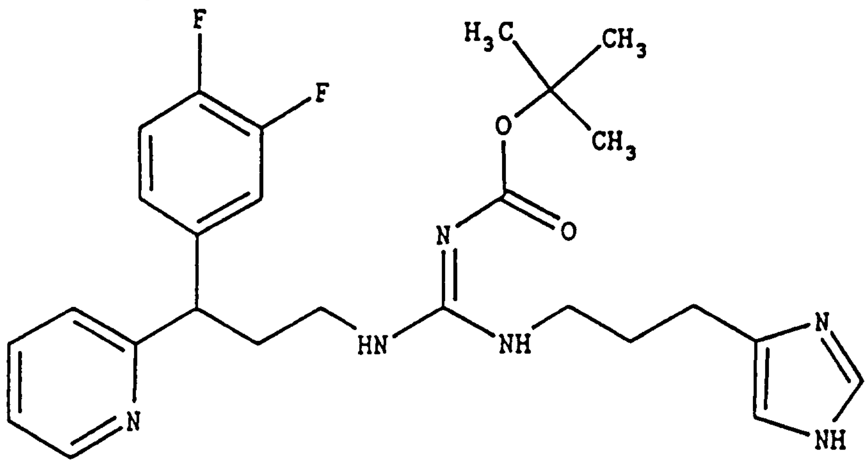

ainsi que ses sels et solvates physiologiquement acceptables.

2. Composé selon la revendication 1, caractérisé en ce que le sel physiologiquement acceptable est un sel d'addition d'acide avec un acide organique ou minéral.

3. Procédé de préparation du composé répondant à la formule I selon la revendication 1, caractérisé en ce que :

   (a) on fait réagir un composé de formule II :

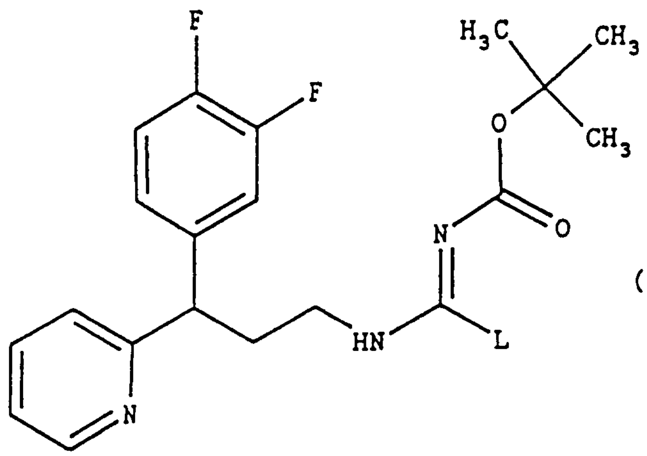

(II)

dans laquelle L représente un groupe alkylthio en $C_1$-$C_4$,alcoxy en $C_1$-$C_4$, arylthio ou aryloxy, avec un composé de formule III :

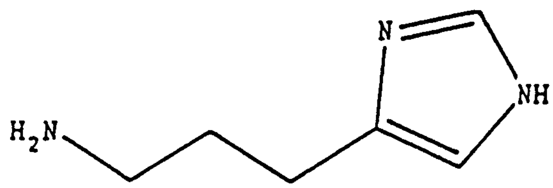

(III)

ou

(b) on fait réagir un composé de formule IV :

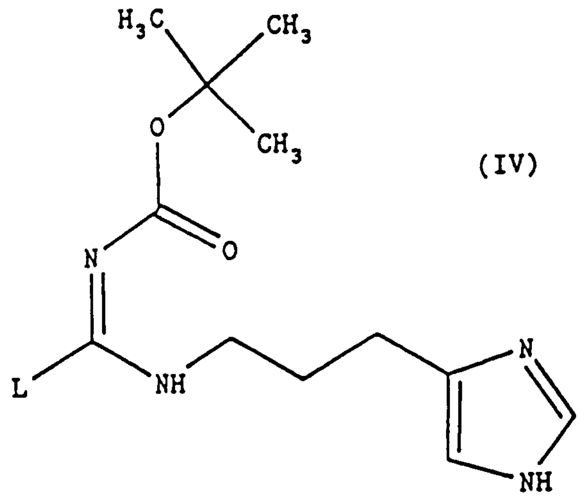

(IV)

dans laquelle L a la signification indiquée ci-dessus, avec un composé de formule V :

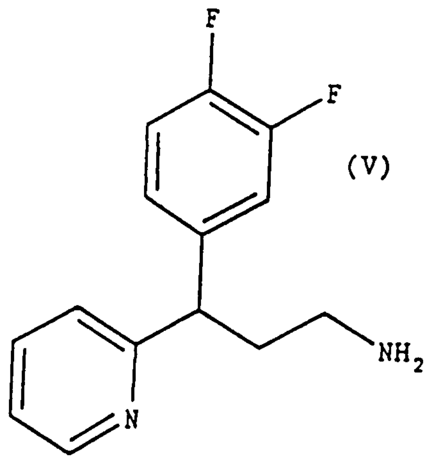

(V)

et, éventuellement, on transforme le composé de formule I obtenu d'après (a) ou (b), d'une manière connue en soi, en son sel ou son solvate physiologiquement acceptable.

**4.** Médicament caractérisé en ce qu'il contient au moins un composé selon la revendication 1 ou 2, conjointement avec un véhicule inerte pharmaceutiquement acceptable ou un diluant inerte pharmaceutiquement acceptable.

**Revendication pour l'Etat contractant suivant : ES**

**1.** Procédé de préparation d'un dérivé d'imidazolylpropylguanidine répondant à la formule I :

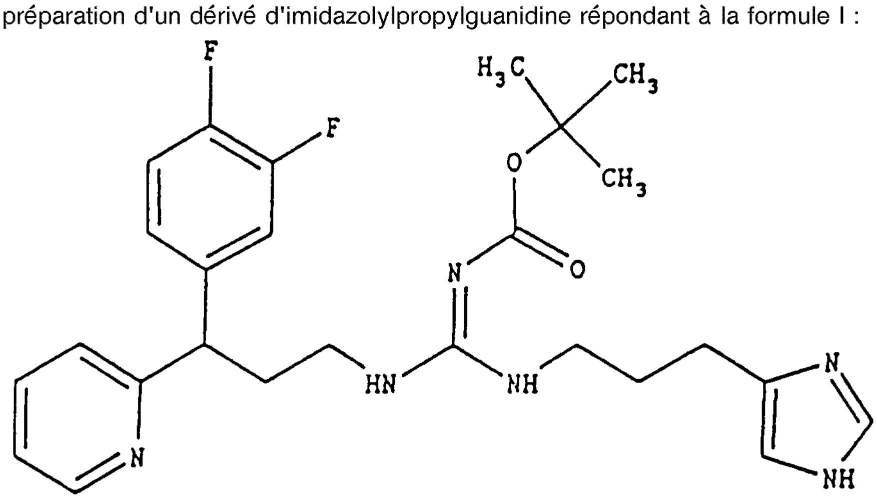

ainsi que de ses sels et solvates physiologiquement acceptables, caractérisé en ce que :

(a) on fait réagir un composé de formule II :

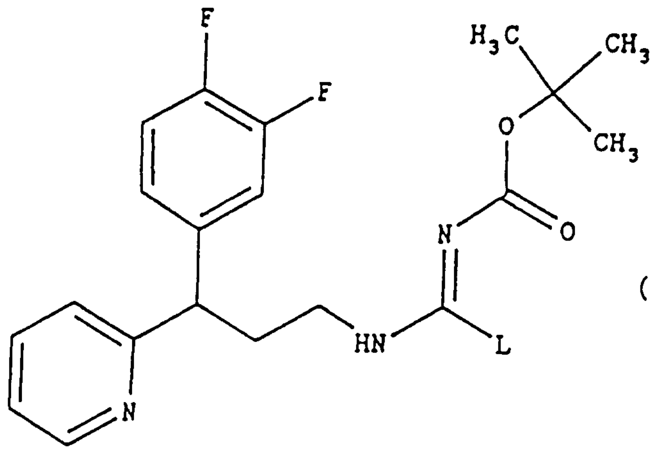

(II)

dans laquelle L représente un groupe alkylthio en $C_1$-$C_4$,alcoxy en $C_1$-$C_4$, arylthio ou aryloxy, avec un composé de formule III :

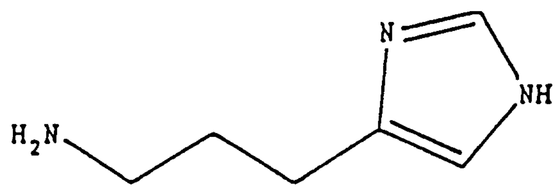

(III)

ou

(b) on fait réagir un composé de formule IV :

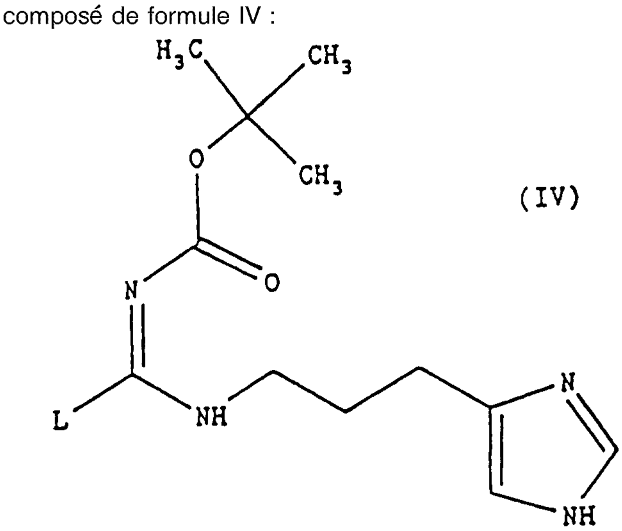

(IV)

dans laquelle L a la signification indiquée ci-dessus, avec un composé de formule V :

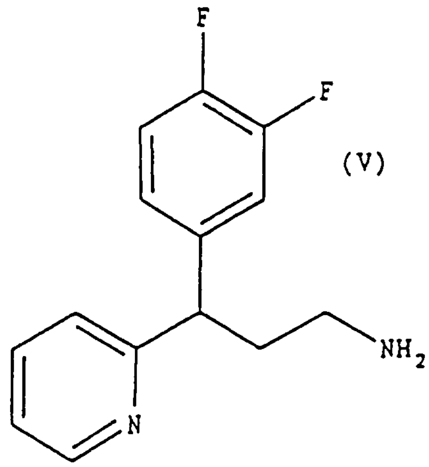

(V)

et, éventuellement, on transforme le composé de formule I obtenu d'après (a) ou (b), d'une manière connue en soi, en son sel ou son solvate physiologiquement acceptable.

**Revendications pour l'Etat contractant suivant : GR**

**1.** Dérivé d'imidazolylpropylguanidine répondant à la formule I :

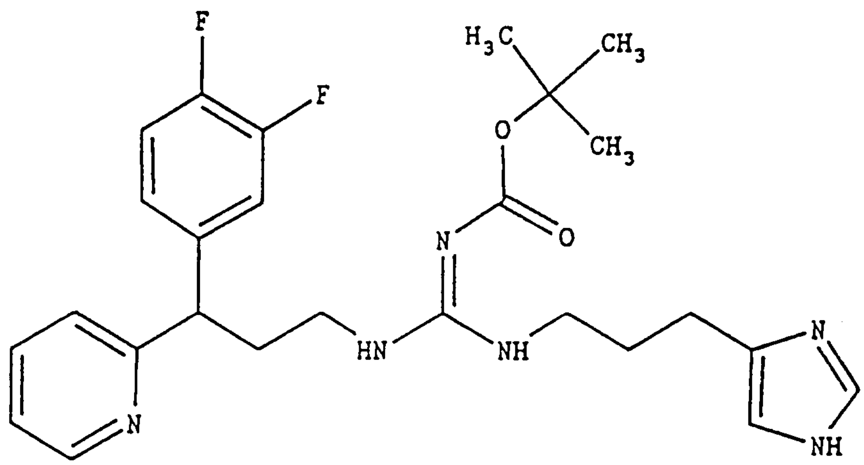

ainsi que ses sels et solvates.

**2.** Composé selon la revendication 1, caractérisé en ce que le sel est un sel d'addition d'acide avec un acide organique ou minéral.

**3.** Procédé de préparation du composé répondant à la formule I selon la revendication 1, caractérisé en ce que :

(a) on fait réagir un composé de formule II :

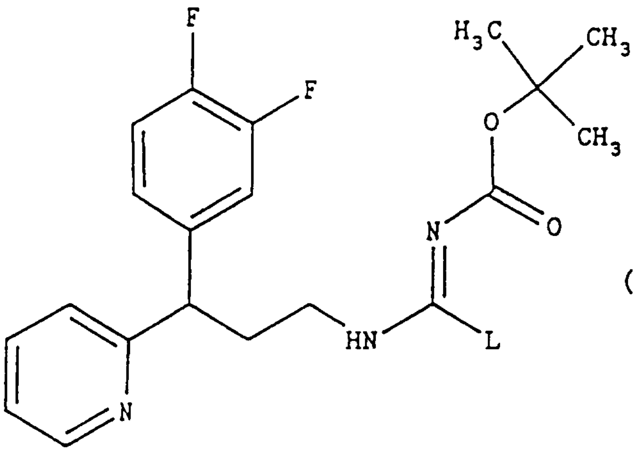

(II)

dans laquelle L représente un groupe alkylthio en $C_1$-$C_4$,alcoxy en $C_1$-$C_4$, arylthio ou aryloxy, avec un composé de formule III :

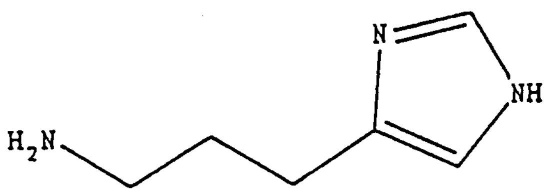

(III)

ou

(b) on fait réagir un composé de formule IV :

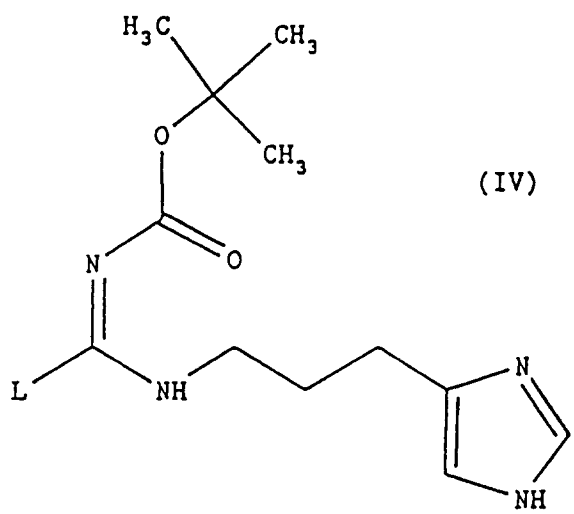

(IV)

dans laquelle L a la signification indiquée ci-dessus, avec un composé de formule V :

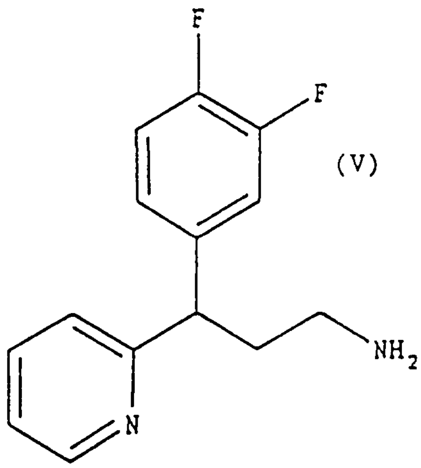

(V)

et, éventuellement, on transforme le composé de formule I obtenu d'après (a) ou (b), d'une manière connue en soi, en son sel ou son solvate physiologiquement acceptable.